(19) Europäisches Patentamt
European Patent Office
Office européen des brevets

(11) **EP 4 613 258 A1**

(12) **EUROPEAN PATENT APPLICATION**
published in accordance with Art. 153(4) EPC

(43) Date of publication:
**10.09.2025 Bulletin 2025/37**

(21) Application number: **23885675.1**

(22) Date of filing: **27.10.2023**

(51) International Patent Classification (IPC):
*A61K 8/34* (2006.01)    *A61Q 1/14* (2006.01)
*C11D 1/66* (2006.01)    *C11D 1/68* (2006.01)
*C11D 3/20* (2006.01)

(52) Cooperative Patent Classification (CPC):
**A61K 8/34; A61Q 1/14; C11D 1/66; C11D 3/20;
C11D 3/2003**

(86) International application number:
**PCT/JP2023/038903**

(87) International publication number:
**WO 2024/095919 (10.05.2024 Gazette 2024/19)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.10.2022 JP 2022175106**

(71) Applicant: **Kao Corporation
Chuo-ku,
Tokyo 103-8210 (JP)**

(72) Inventors:
• **UEYAMA, Chihiro
  Tokyo 131-8501 (JP)**
• **TSUDA, Hiroko
  Tokyo 131-8501 (JP)**
• **KAGAYA, Mariko
  Tokyo 131-8501 (JP)**
• **NAGASAKI, Yuko
  Tokyo 131-8501 (JP)**

(74) Representative: **Hoffmann Eitle
Patent- und Rechtsanwälte PartmbB
Arabellastraße 30
81925 München (DE)**

(54) **CLEANING AGENT COMPOSITION**

(57) A cleansing composition including the following components (A), (B) and (C): (A) from 9 to 50% by mass of a nonionic surfactant including: (a1) a nonionic surfactant having an HLB value of 8.5 or more and less than 12.5, (a2) a nonionic surfactant having an HLB value of 12.5 or more and 18 or less, and (a3) a nonionic surfactant having an HLB value of 1.8 or more and less than 8.5, wherein a mass ratio of the component (a2) to the total amount of the components (a1) and (a3), (a2)/[(a1)+(a3)], is from 0.5 to 2.5, (B) from 45 to 90% by mass of an oil phase having a viscosity at 25°C of 12.0 mPa·s or less, and (C) from 0.05 to 15% by mass of one or more selected from the group consisting of water, an alcohol having 2 to 3 carbon atoms and a polyol, wherein a mass ratio of the component (C) to the component (A), (C)/(A) is 0.40 or less.

EP 4 613 258 A1

**Description**

Field of the Invention

The present invention relates to a cleansing composition.

Background of the Invention

In makeup remover, one of the important performance requirements of consumers is high cleansing power to remove long-lasting makeup. Usually, when using makeup remover, a method used is to remove makeup by mixing the remover and makeup through hand massage (friction). Oil-type makeup removers are a formulation provided by combining a number of oils that provide cleansing power with surfactant that emulsifies the oils for rinsing, and are widely accepted in the market. Meanwhile, there are issues such as unique use impression of the oil, and many studies are being conducted to improve these issues.

For example, Patent Literature 1 discloses a cleansing composition containing a specific amount of high average HLB nonionic surfactant, a specific alkyl monoester, a hydrocarbon oil, a polyol, and water, which has excellent skin feel and high rinse speed.

Patent Literature 2 describes that a cleansing composition which contains three specific nonionic surfactants, an oil agent, a water-soluble solvent and water can be cleanly washed off without leaving residual feeling, gives no oily feeling, can quickly lift out hard-to-remove mascara and other residues, and has good stability.

Patent Literature 3 discloses that a transparent oily cleansing cosmetic which includes a specific oil component containing an ester group in the molecule, a non-polar hydrocarbon oil, a volatile silicone oil, a polyoxyethylene fatty acid glycerol and water removes makeup well, has fresh feeling of rinsing, and does not become cloudy or thicken significantly due to emulsification or suspension even if a significant amount of water is mixed in.

(Patent Literature 1) JP-A-2018-508484
(Patent Literature 2) JP-A-2008-184415
(Patent Literature 3) JP-A-2009-235001

Summary of the Invention

The present invention relates to a cleansing composition comprising the following components (A), (B) and (C):

(A) from 9 to 50% by mass of a nonionic surfactant comprising:

(a1) a nonionic surfactant having an HLB value of 8.5 or more and less than 12.5,
(a2) a nonionic surfactant having an HLB value of 12.5 or more and 18 or less, and
(a3) a nonionic surfactant having an HLB value of 1.8 or more and less than 8.5,
wherein a mass ratio of the component (a2) to the total amount of the components (a1) and (a3), (a2)/[(a1)+(a3)], is from 0.5 to 2.5,

(B) from 45 to 90% by mass of an oil phase having a viscosity at 25°C of 12.0 mPa·s or less, and
(C) from 0.05 to 15% by mass of one or more selected from the group consisting of water, an alcohol having 2 to 3 carbon atoms and a polyol,

wherein a mass ratio of the component (C) to the component (A), (C)/(A) is 0.4 or less.

Detailed Description of the Invention

In recent years, there has been an increasing trend of consumer awareness wishing to take care of their skin without rubbing it. In addition, consumer behavior has shifted toward low-friction, short-time use of makeup remover to remove makeup in busy daily lives. Under such circumstances, long-lasting foundation sometimes remains even when using oil-type makeup remover, which is said to have high cleansing power, and this has been one of the user's complaints. Furthermore, when the makeup remover composition itself was poorly rinsed, the short-time, low-friction rinsing action left makeup remover on the skin, and thus it was necessary to take time to rinse the skin with water with some friction.

Meanwhile, with global warming and living with masks in infectious disease epidemics, highly long-lasting foundations that do not easily come off are now preferred. For example, a technique is generally used, in which an oil-soluble silicone resin such as trimethylsiloxysilicate (TMS) is blended in a long-lasting foundation to form a skin-following, water-repellent makeup coating film on the skin surface to not only achieve sweat sebum resistance but also physically prevent makeup from smudging.

Oil is useful for cleansing oil-soluble silicone resin and hydrophobic powder blended in a long-lasting foundation, but if the composition is made only with surfactants that dissolve easily in oil, they will not blend well with water, resulting in poor rinsing properties. On the other hand, if a certain amount of hydrophilic surfactant is added to increase rinsing properties, the composition will not be able to maintain a one-layer state at a certain temperature (failing to maintain a desirable appearance).

Thus, with conventional compositions, it was difficult to achieve all of new needs arising from the changing times including low-friction removal of long-lasting foundation, good rinsing properties and a desirable appearance.

The present inventors found that a cleansing composition combining three kinds of nonionic surfactants with different HLBs, water or a polyol and the like, and low viscosity oil in a specific proportion can achieve all of cleansing power to remove long-lasting foundation with little friction, good rinsing properties, and a desirable appearance of the composition.

The cleansing composition of the present invention achieves all of the high cleansing power to remove hard-to-remove long-lasting foundation containing film-forming agent with little physical force, good rinsing properties, and a desirable appearance of the composition.

The component (A) is a nonionic surfactant comprising:

(a1) a nonionic surfactant having an HLB value of 8.5 or more and less than 12.5,
(a2) a nonionic surfactant having an HLB value of 12.5 or more and 18 or less, and
(a3) a nonionic surfactant having an HLB value of 1.8 or more and less than 8.5.

As used herein, the HLB value is an indicator of hydrophile-lipophile balance. In the present description, HLB values are calculated by the following Griffin formula.

$$HLB = 20 \times \frac{\text{Molecular weight of hydrophilic moiety}}{\text{Molecular weight of surfactant}}$$

When two or more kinds of nonionic surfactants are included, the HLB value of the mixed surfactant is the arithmetic mean of the HLB value of the respective nonionic surfactants based on their mass ratio.

$$\text{Mixed HLB} = \Sigma(HLB_x \times W_x) / \Sigma W_x$$

$HLB_x$ denotes the HLB value of nonionic surfactant X.

$W_x$ denotes the mass (g) of nonionic surfactant X with a value of $HLB_x$.

The component (a1) is a nonionic surfactant having an HLB value of 8.5 or more and less than 12.5.

The component (a1) preferably comprises one or more selected from the group consisting of a POE sorbitan fatty acid ester, a POE sorbitol fatty acid ester, a POE glycerol fatty acid ester and a POE fatty acid ester, and preferably comprises a multi-chain type, and more preferably comprises one or more selected from the group consisting of a multi-chain POE sorbitan fatty acid ester and a multi-chain POE sorbitol fatty acid ester from the viewpoint of cleansing properties.

The content of the component (a1) is preferably 2% by mass or more, and more preferably 3% by mass or more, and preferably 20% by mass or less, and more preferably 15% by mass or less in the entire composition from the viewpoint of excellent cleansing power and a desirable appearance. The content of the component (a1) is preferably from 2 to 20% by mass, and more preferably from 3 to 15% by mass in the entire composition.

The component (a2) is a nonionic surfactant having an HLB value of 12.5 or more and 18 or less.

The component (a2) preferably comprises one or more selected from the group consisting of a POE fatty acid ester, a POE alkyl ether, a POE sorbitol fatty acid ester, a POE glycerol fatty acid ester, a polyglycerol fatty acid ester and an alkyl glucoside, and more preferably comprises one or more selected from the group consisting of a POE fatty acid ester, a POE glycerol fatty acid ester and an alkyl glucoside from the viewpoint of cleansing properties.

The content of the component (a2) is preferably 5% by mass or more, and more preferably 7% by mass or more, and preferably 30% by mass or less, and more preferably 25% by mass or less in the entire composition from the viewpoint of excellent cleansing power, a desirable appearance and rinsing properties. The content of the component (a2) is preferably from 5 to 30% by mass, and more preferably from 7 to 25% by mass in the entire composition.

The component (a3) is a nonionic surfactant having an HLB value of 1.8 or more and less than 8.5.

The component (a3) preferably comprises one or more selected from the group consisting of a glycerol fatty acid ester, a polyglycerol fatty acid ester, an alkyl glyceryl ether and a sorbitan fatty acid ester, and preferably comprises those with a glycerol backbone, and more preferably comprises an ester with a glycerol backbone from the viewpoint of cleansing properties.

The content of the component (a3) is preferably 1% by mass or more, and more preferably 3% by mass or more, and preferably 15% by mass or less, and more preferably 10% by mass or less in the entire composition from the viewpoint of a desirable appearance. The content of the component (a3) is preferably from 1 to 15% by mass, and more preferably from 3 to 10% by mass in the entire composition.

In the present invention, the mass ratio of the component (a2) to the total amount of the components (a1) and (a3), (a2)/[(a1)+(a3)], is 0.5 or more, preferably 0.8 or more, and more preferably 1 or more, and 2.5 or less, preferably 2 or less, more preferably 1.7 or less, and further preferably 1.5 or less from the viewpoint of excellent rinsing properties, a desirable appearance and cleansing power. The mass ratio of the component (a2) to the total amount of the components (a1) and (a3), (a2)/[(a1)+(a3)], is from 0.5 to 2.5, preferably from 0.8 to 2, more preferably from 1 to 1.7, and further preferably from 1 to 1.5.

The component (A) comprises the above components (a1), (a2) and (a3), and has a mixed HLB value of preferably from 10 to 13, and more preferably from 10.5 to 12.5 from the viewpoint of cleansing power, rinsing properties and a desirable appearance.

The content of the component (A) is preferably 9% by mass or more, more preferably 10% by mass or more, further preferably 15% by mass or more, and even more preferably 20% by mass or more in the entire composition, and 50% by mass or less, preferably 45% by mass or less, more preferably 40% by mass or less, and further preferably 35% by mass or less in the entire composition from the viewpoint of excellent cleansing power and a desirable appearance. The content of the component (A) is from 9 to 50% by mass, preferably from 10 to 45% by mass, more preferably from 15 to 40% by mass, and further preferably from 20 to 35% by mass in the entire composition.

The component (B) is an oil phase having a viscosity at 25°C of 12.0 mPa·s or less.

The oil phase of the component (B) has the function of dissolving the oil-soluble polymer that forms a hard film on the surface of the long-lasting foundation film and delivering the component (A) into the long-lasting foundation film. From that point of view, the oil phase has a viscosity at 25°C of 12.0 mPa·s or less, preferably 10 mPa·s or less, and more preferably 9 mPa·s or less.

The viscosity is measured by using a B-type viscometer (TVB-10 viscometer manufactured by Toki Sangyo Co., Ltd.), and a viscosity less than 100 mPa·s at 25°C is measured with a rotor No. 1 at a rotation number of 60 rpm in 1 minute of measurement time.

One component (B) may be used, or two or more of them may be used in combination. When the oil phase is composed of two or more oils, a value obtained by calculating the arithmetic mean of the viscosity of each oil based on their mass ratio is determined as the viscosity.

The oil constituting the oil phase of the component (B) is those used in usual cleansing compositions, and examples thereof include hydrocarbon oil, ester oil, ether oil and silicone oil.

Specific examples of hydrocarbon oils include isododecane, isohexadecane, light liquid isoparaffin, liquid isoparaffin and liquid paraffin.

Examples of ester oils include a monoester of an aliphatic or aromatic monocarboxylic acid or dicarboxylic acid having 2 to 24 carbon atoms, which are specifically cetyl 2-ethylhexanoate, cetyl octanonate, isononyl isononanoate, isotridecyl isononanoate, hexyl laurate, isopropyl myristate, octyldodecyl myristate, myristyl myristate, 2-hexyldecyl myristate, isopropyl palmitate, octyl palmitate, propylene glycol dicaprylate, neopentyl glycol dicaprate, caprylic/capric triglyceride, glyceryl trioleate, glyceryl tri(2-ethylhexanoate), olive oil and jojoba oil.

Examples of ether oils include a dialkyl ether, which are specifically dihexyl ether, dicaprylyl ether and cetyl-1,3-dimethylbutyl ether.

Examples of silicone oils include volatile or nonvolatile linear dimethyl polysiloxane such as dimethyl polysiloxane (1 cs), dimethyl polysiloxane (1.5 cs), dimethyl polysiloxane (2 cs) and dimethyl polysiloxane (6 cs).

It is preferable that no silicone oil is included (0%) from the viewpoint of a desirable appearance.

It is preferable to use a non-polar oil and a polar oil in combination as the component (B) to achieve a desirable appearance and to develop cleansing properties regardless of the combination of surfactants.

When a non-polar oil and a polar oil are used in combination, the mass ratio of the non-polar oil to the polar oil (non-polar oil/ polar oil) is preferably from 0.1 to 9, and more preferably from 0.17 to 6.

Furthermore, it is preferable to include 70% by mass or more of one or more selected from the group consisting of:

(b1) one or more non-polar oils selected from the group consisting of isododecane, isohexadecane, hydrogenated polyisobutene and light liquid isoparaffin; and
(b2) a polar oil having a viscosity at 25°C of 10 mPa·s or less from the viewpoint of a desirable appearance and cleansing properties.

When the component (b1) and the component (b2) are used in combination, the mass ratio of the component (b1) to the component (b2), (b1)/(b2), is preferably from 0.1 to 9, and more preferably from 0.17 to 6.

The content of the component (B) is 45% by mass or more, preferably 50% by mass or more and more preferably 60% by mass or more, and 90% by mass or less, and preferably 80% by mass or less in the entire composition so as to quickly dissolve the film forming agent to develop high cleansing power, and from the viewpoint of a desirable appearance. The content of the component (B) is from 45 to 90% by mass, preferably from 50 to 90% by mass, and more preferably from 60 to 80% by mass in the entire composition.

The component (C) is one or more selected from the group consisting of water, an alcohol having 2 to 3 carbon atoms and a polyol. The component (C) enhances the dissolution stability of the component (A) in the system and at the same time activates the function of surfactant to assist in cleansing power.

Examples of an alcohol having 2 or 3 carbon atoms include ethanol and isopropyl alcohol.

Divalent alcohol and trivalent alcohol are preferred as polyol, and divalent alcohol is more preferred.

Examples of divalent alcohol include ethylene glycol, diethylene glycol, triethylene glycol, polyethylene glycol, propylene glycol, dipropylene glycol, tripropylene glycol, polypropylene glycol, butylene glycol and propanediol.

Examples of trivalent alcohol include glycerol.

Water, ethanol, 1,3-butylene glycol and dipropylene glycol are preferred as the component (C). It is preferable to include water from the viewpoint of the appearance of the composition.

One component (C) may be used, or two or more of them may be used in combination. The content of the component (C) is 0.05% by mass or more, preferably 0.5% by mass or more, and more preferably 1.5% by mass or more, and 15% by mass or less, preferably 8% by mass or less, and more preferably 7.5% by mass or less in the entire composition from the viewpoint of a desirable appearance and cleansing power. The content of the component (C) is from 0.05 to 15% by mass, preferably from 0.5 to 8% by mass, and more preferably from 1.5 to 7.5% by mass in the entire composition.

In the present invention, the mass ratio of the component (C) to the component (A), (C)/(A), is 0.40 or less, preferably 0.35 or less, and more preferably 0.30 or less, and preferably 0.02 or more, more preferably 0.05 or more, and further preferably 0.15 or more from the viewpoint of cleansing power and a desirable appearance. The mass ratio of the component (C) to the component (A), (C)/(A), is 0.40 or less, preferably from 0.02 to 0.40, more preferably from 0.05 to 0.35, and further preferably from 0.15 to 0.30.

The cleansing composition of the present invention may also include a component used in ordinary cleansing compositions, such as a surfactant other than the component (A), an oil component other than the component (B), a polymer compound, an ultraviolet absorber, an antioxidant, a perfume, an antiseptic, a preservative and an antifouling agent in addition to the above components.

The cleansing composition of the present invention may be produced by heating and dissolving components which is solid at room temperature, and then mixing and stirring the respective components by a usual method.

The cleansing composition of the present invention is an oil formulation, and preferably has the appearance of a one-layer-transparent solution from the aesthetic point of view that is typical of oils, and as the composition is uniform and can provide constant performance every time.

Transparent means a transparent or slightly translucent appearance, and when the composition is placed in a glass container (Screw tube bottle No. 5 manufactured by Maruem) and then the product barcode is placed at a position 3 cm from the back of the container, the barcode numbers are observable when viewed from the front of the glass container containing the composition.

The cleansing composition of the present invention is preferably in the form of liquid for ease of dispensing when filled into a pump dispenser to be used.

Liquid means that it can be easily spilled when held in the palm of the hand and tilted. The cleansing composition has a viscosity measured using a B-type viscometer (TVB-10 viscometer manufactured by Toki Sangyo) at 25°C of preferably 200 mPa·s or less, and more preferably 100 mPa·s of less.

The cleansing composition of the present invention, when blended into makeup as a makeup remover, removes long-lasting foundation without the need for scrubbing as conventional, and because the makeup remover itself has good rinsing properties, makeup removal can be completed without scrubbing when rinsing. Compared to conventional products, the present invention enables a reduction in friction on the skin during both blending and rinsing of makeup remover, i.e., during the entire makeup remover action.

It is also possible to remove makeup almost without touching the skin by filling a mist dispenser or aerosol container with the cleansing composition of the present invention, spraying it in the form of mist onto the face , and rinsing it off in the shower by applying water directly to the face.

The cleansing composition of the present invention is preferably used for skin cleansing, especially as a face wash, a makeup remover, and a body wash for removing oily stains such as sunscreen.

For the above embodiments, the present invention also discloses the following cleansing compositions.

<1> A cleansing composition comprising the following components (A), (B) and (C):

(A) from 9 to 50% by mass of a nonionic surfactant comprising

(a1) a nonionic surfactant having an HLB value of 8.5 or more and less than 12.5,
(a2) a nonionic surfactant having an HLB value of 12.5 or more and 18 or less, and
(a3) a nonionic surfactant having an HLB value of 1.8 or more and less than 8.5,
wherein a mass ratio of the component (a2) to the total amount of the components (a1) and (a3), (a2)/[(a1)+(a3)], is from 0.5 to 2.5,

(B) from 45 to 90% by mass of an oil phase having a viscosity at 25°C of 12.0 mPa·s or less, and
(C) from 0.05 to 15% by mass of one or more selected from the group consisting of water, alcohol having 2 to 3 carbon atoms and polyol,

wherein the mass ratio of the component (C) to the component (A), (C)/(A) is 0.40 or less.

<2> The cleansing composition according to <1> above, wherein the component (A) has a mixed HLB value of from 10 to 13, and preferably from 10.5 to 12.5.

<3> The cleansing composition according to <1> or <2> above, wherein a content of the component (A) is preferably from 10 to 45% by mass, more preferably from 15 to 40% by mass, and further preferably from 20 to 35% by mass in the entire composition.

<4> The cleansing composition according to any one of <1> to <3> above, wherein a mass ratio of the component (a2) to the total amount of the components (a1) and (a3), (a2)/[(a1)+(a3)], is preferably from 0.8 to 2, more preferably from 1 to 1.7, and further preferably from 1 to 1.5.

<5> The cleansing composition according to any one of <1> to <4> above, wherein a content of the component (a1) is preferably from 2 to 20% by mass, and more preferably from 3 to 15% by mass in the entire composition.

<6> The cleansing composition according to any one of <1> to <5> above, wherein the component (a1) is one or more selected from the group consisting of a POE sorbitan fatty acid ester, a POE sorbitol fatty acid ester, a POE glycerol fatty acid ester and a POE fatty acid ester.

<7> The cleansing composition according to any one of <1> to <6> above, wherein the component (a1) preferably comprises a multi-chain type, and more preferably comprises one or more selected from the group consisting of a multi-chain POE sorbitan fatty acid ester and a multi-chain POE sorbitol fatty acid ester.

<8> The cleansing composition according to any one of <1> to <7> above, wherein a content of the component (a2) is preferably from 5 to 30% by mass, and more preferably from 7 to 25% by mass in the entire composition.

<9> The cleansing composition according to any one of <1> to <8> above, wherein the component (a2) is one or more selected from the group consisting of a POE fatty acid ester, a POE alkyl ether, a POE sorbitol fatty acid ester, a POE glycerol fatty acid ester, a polyglycerol fatty acid ester and an alkyl glucoside.

<10> The cleansing composition according to any one of <1> to <9> above, wherein the component (a2) comprises one or more selected from the group consisting of a POE fatty acid ester, a POE glycerol fatty acid ester and an alkyl glucoside.

<11> The cleansing composition according to any one of <1> to <10> above, wherein a content of the component (a3) is preferably from 1 to 15% by mass, and more preferably from 3 to 10% by mass in the entire composition.

<12> The cleansing composition according to any one of <1> to <11> above, wherein the component (a3) is one or more selected from the group consisting of a glycerol fatty acid ester, a polyglycerol fatty acid ester, an alkyl glyceryl ether and a sorbitan fatty acid ester.

<13> The cleansing composition according to any one of <1> to <12> above, wherein the component (a3) preferably comprises those with a glycerol backbone, and more preferably comprises an ester with a glycerol backbone.

<14> The cleansing composition according to any one of <1> to <13> above, wherein the component (B) has a viscosity at 25°C of preferably 10 mPa·s or less, and more preferably 9 mPa·s or less.

<15> The cleansing composition according to any one of <1> to <14> above, wherein a content of the component (B) is preferably from 50 to 90% by mass, and more preferably from 60 to 80% by mass in the entire composition.

<16> The cleansing composition according to any one of <1> to <15> above, wherein the component (B) comprises one or more selected from the group consisting of a hydrocarbon oil, an ester oil, an ether oil and a silicone oil.

<17> The cleansing composition according to any one of <1> to <16> above, wherein the component (B) does not include a silicone oil.

<18> The cleansing composition according to any one of <1> to <17> above, wherein the component (B) comprises a non-polar oil and a polar oil.

<19> The cleansing composition according to any one of <1> to <18> above, wherein a mass ratio of the non-polar oil to the polar oil (non-polar oil/ polar oil) is preferably from 0.1 to 9, and more preferably from 0.17 to 6.

<20> The cleansing composition according to any one of <1> to <19> above, wherein the component (B) comprises 70% by mass or more of one or more selected from the group consisting of:

(b1) one or more non-polar oils selected from the group consisting of isododecane, isohexadecane, hydrogenated polyisobutene and light liquid isoparaffin; and
(b2) a polar oil having a viscosity at 25°C of 10 mPa·s or less.

<21> The cleansing composition according to <20> above, wherein a mass ratio of the component (b1) to the component (b2), (b1)/(b2), is from 0.1 to 9, and preferably from 0.17 to 6.

<22> The cleansing composition according to any one of <1> to <21> above, wherein the component (C) is one or more selected from the group consisting of water, ethanol, 1,3-butylene glycol and dipropylene glycol, and the component (C) preferably comprises water.

<23> The cleansing composition according to any one of <1> to <22> above, wherein a content of the component (C) is preferably from 0.5 to 8% by mass, and more preferably from 1.5 to 7.5% by mass in the entire composition.

<24> The cleansing composition according to any one of <1> to <23> above, wherein a mass ratio of the component (C) to the component (A), (C)/(A) is preferably from 0.02 to 0.40, more preferably from 0.05 to 0.35, and further preferably from 0.15 to 0.30.

<25> The cleansing composition according to any one of <1> to <24> above, wherein the cleansing composition is preferably for skin cleansing, is more preferably a face wash, a makeup remover or a body wash.

<26> The cleansing composition according to any one of <1> to <25> above, wherein a container with a mist dispenser or an aerosol container is filled with the cleansing composition and the cleansing composition is used by spraying to the skin.

Examples

Examples 1 to 57, Comparative Examples 1 to 18

Cleansing compositions with the composition shown in Table 1 to Table 9 were produced, and the appearance of the composition (immediately after production, 25°C), the appearance of the composition (50°C), the appearance of the composition (-5°C), the cleansing power and the rinsing properties were evaluated. The results are shown together in Table 1 to Table 5.

(Production method)

The component (A) is weighed and mixed well. Then, the component (B) was added thereto with stirring, and finally the component (C) was added dropwise thereto and the mixture was stirred until homogeneous to give a cleansing composition.

(Evaluation method)

(1) Appearance of composition (immediately after production, 25°C):

The appearance of the respective compositions immediately after production was visually evaluated according to the following criteria.

3; Transparent (transparent to slightly translucent appearance; when the composition is placed in a glass container (Screw tube bottle No. 5 manufactured by Maruem) and then the product barcode is placed at a position 3 cm from the back of the container, the barcode numbers are observable when viewed from the front of the glass container containing the composition).

2; Translucent (translucent appearance; when the composition is placed in a glass container (Screw tube bottle No. 5 manufactured by Maruem) and then the product barcode is placed at a position 3 cm from the back of the container, the barcode numbers are not observable when viewed from the front of the glass container containing the composition, while when the product barcode is placed in contact with the back of the container, the barcode numbers are observable when viewed from the front of the container).

1; Opaque (opaque appearance; when the composition is placed in a glass container (Screw tube bottle No. 5 manufactured by Maruem) and then the product barcode is placed in contact with the back of the container, the barcode numbers are not observable when viewed from the front of the container. In the case of this state, the composition separates into two layers the next day).

A rating of "2" or higher is considered pass.

(2) Appearance of composition (50°C):

The respective cleansing compositions were stored at 50°C for 24 hours and then the appearance of the compositions was visually evaluated according to the following criteria. The criteria for transparent, translucent, and opaque are the same as in (1).

> 3; Transparent.
> 2; Translucent.
> 1; Opaque.

(3) Appearance of composition (-5°C):

The respective cleansing compositions were stored at -5°C for 24 hours and then the appearance of the compositions was visually evaluated according to the following criteria. The criteria for transparent, translucent, and opaque are the same as in (1).

> 3; Transparent.
> 2; Translucent.
> 1; Opaque.

(4) Cleansing power:

A long-lasting foundation (Revlon Colorstay Makeup Foundation) is applied to a model sheet with pore-like small dents (artificial leather Laforet White made by OkamotoKaseihin Co. Ltd.) at 0.065 mg/cm$^2$, and dried for 1 hour or more.
The foundation applied site is cut into small pieces of $2 \times 2.5$ cm$^2$ to make a test piece.

(4-1) Cleansing power when 100 g of friction force is applied:

One drop of each cleansing composition was dropped onto the test piece with a syringe and the test piece was massaged three times with the index finger in a circular motion with a 100 g load. The foundation was then rinsed off in the shower, dried, and visually evaluated for removal according to the following criteria.
A rating of "3" or higher is considered pass.

> 1; Foundation came off in less than 20% of the area touched by the hand.
> 2; Foundation came off in 20% or more of the area touched by the hand.
> 3; Foundation came off in 40% or more of the area touched by the hand.
> 4; Foundation came off in 60% or more of the area touched by the hand.
> 5; Foundation came off in 80% or more of the area touched by the hand.

(4-2) Cleansing power when 3 g of friction force is applied:

One drop of each cleansing composition was dropped onto the test piece with a syringe and the test piece was massaged three times with the index finger in a circular motion with a 3 g load. The foundation was then rinsed off in the shower, dried, and visually evaluated for removal according to the following criteria.
A rating of "3" or higher is considered pass.

> 1; Foundation came off in less than 20% of the area touched by the hand.
> 2; Foundation came off in 20% or more of the area touched by the hand.
> 3; Foundation came off in 30% or more of the area touched by the hand.
> 4; Foundation came off in 40% or more of the area touched by the hand.
> 5; Foundation came off in 50% or more of the area touched by the hand.

(4-3) Cleansing power without friction force:

One drop of each cleansing composition was dropped onto the test piece with a syringe to allow the composition to be in contact with the coating film of the foundation for 10 seconds. The foundation was then rinsed off in the shower, dried, and visually evaluated for removal according to the following criteria.

1; Foundation came off in less than 20% of the area in contact with the composition.

2; Foundation came off in 20% or more of the area in contact with the composition.

3; Foundation came off in 40% or more of the area in contact with the composition.

4; Foundation came off in 60% or more of the area in contact with the composition.

5; Foundation came off in 80% or more of the area in contact with the composition.

(5) Rinsing properties:

1.5 g of each cleansing composition was gently spread over the entire face, gently pressed with the palm of the hand, and then rinsed with water. The speed of rinsing was evaluated.

A rating of "2" or higher is considered pass.

1; Rinsed off slowly.

2; Rinsed off slightly quickly.

3; Rinsed off quickly.

[Table 1]

| | Component name | Raw material name | Example | | | | | | | | |
|---|---|---|---|---|---|---|---|---|---|---|---|
| | | | 1 | 2 | 3 | 4 | 5 | 6 | 7 | 8 | 9 |
| A(a1) | Polyoxyethylene (30) sorbitol tetra-oleate | HLB10.5: RHEO-DOL 430 (manu-factured by Kao Corporation) | | 6 | | | | | 3 | 3 | 3 |
| | Polyoxyethylene (20) sorbitan trioleate | HLB11: RHEODOL TW-O320V (manufactured by Kao Corporation) | 5 | | | | | | | | |
| | Polyoxyethylene (6) sorbitan stea-rate | HLB9.6: RHEO-DOL TW-S106V (manufactured by Kao Corporation) | | | 6 | | | | 3 | | |
| | Polyoxyethylene (6) sorbitan oleate | HLB10: RHEODOL TW-O106V (manufactured by Kao Corporation) | | | | 6 | | | | 3 | |
| | Polyoxyethylene (10) glyceryl isostearate | HLB10: EMALEX GWIS-110EX (manufactured by Nihon Emulsion Co., Ltd.) | | | | | | 6 | | | 3 |
| | Polyoxyethylene (8) glyceryl isostearate | HLB10 :EMALEX GWIS-108 (man-ufactured by Ni-hon Emulsion Co., Ltd.) | | | | | | | 6 | | |
| A(a2) | Polyoxyethylene (12) lauric acid ester | HLB14: EMANON 1112 (manufac-tured by Kao Cor-poration) | 10 | 10 | 10 | 10 | 10 | 10 | 10 | 10 | 10 |
| | Alkyl glucoside | HLB18: AG-10LK (manufactured by Kao Corporation) | 3 | 3 | 3 | 3 | 3 | 3 | 3 | 3 | 3 |

(continued)

| | | Component name | Raw material name | Example | | | | | | | | |
|---|---|---|---|---|---|---|---|---|---|---|---|---|
| | | | | 1 | 2 | 3 | 4 | 5 | 6 | 7 | 8 | 9 |
| A(a3) | | Polyglyceryl-2 isostearate | HLB6: COSMOL 41V (manufactured by The Nisshin Oillio Group, Ltd.) | 5 | 6 | 6 | 6 | 6 | 6 | 6 | 6 | 6 |
| B | | Light liquid isoparaffin | PARLEAM 4 (manufactured by NOF Corporation) | 24.3 | 23.5 | 23.5 | 23.5 | 23.5 | 23.5 | 23.5 | 23.5 | 23.5 |
| | | Isononyl isononanoate | SALACOS 99 (manufactured by The Nisshin Oillio Group, Ltd.) | 24.3 | 23.5 | 23.5 | 23.5 | 23.5 | 23.5 | 23.5 | 23.5 | 23.5 |
| | | Isopropyl myristate | EXCEPARL IPM (manufactured by Kao Corporation) | 24.3 | 23.5 | 23.5 | 23.5 | 23.5 | 23.5 | 23.5 | 23.5 | 23.5 |
| C | | Water | | 4 | 4.5 | 4.5 | 4.5 | 4.5 | 4.5 | 4.5 | 4.5 | 4.5 |
| | | Total | | 100 | 100 | 100 | 100 | 100 | 100 | 100 | 100 | 100 |
| | | Mixed HLB of component (A) | | 11.3 | 11.7 | 11.5 | 11.6 | 11.6 | 11.6 | 11.6 | 11.7 | 11.7 |
| | | (a2)/[(a1)+(a3)] | | 1.0 | 1.1 | 1.1 | 1.1 | 1.1 | 1.1 | 1.1 | 1.1 | 1.1 |
| | | Total amount of component (A) | | 23 | 25 | 25 | 25 | 25 | 25 | 25 | 25 | 25 |
| | | Total amount of oil phase (B) | | 73.0 | 70.5 | 70.5 | 70.5 | 70.5 | 70.5 | 70.5 | 70.5 | 70.5 |
| | | Arithmetic mean of viscosity of oil phase (B) | | 6.3 | 6.5 | 6.3 | 6.3 | 6.3 | 6.3 | 6.3 | 6.3 | 6.3 |
| | | C/A | | 0.17 | 0.18 | 0.18 | 0.18 | 0.18 | 0.18 | 0.18 | 0.18 | 0.18 |
| | | Appearance of composition (immediately after production, 25°C) | | 3 | 3 | 3 | 3 | 3 | 3 | 3 | 3 | 3 |
| | | Appearance of composition (50°C) | | 3 | 3 | 3 | 3 | 3 | 3 | 3 | 3 | 3 |
| | | Appearance of composition (-5°C) | | 3 | 3 | 2 | 3 | 3 | 3 | 2 | 3 | 3 |
| | | Cleansing power when 100 g of friction force is applied | | 5 | 5 | 5 | 5 | 5 | 5 | 5 | 5 | 5 |
| | | Cleansing power when 3 g of friction force is applied | | 5 | 5 | 5 | 5 | 5 | 5 | 5 | 5 | 5 |
| | | Rinsing properties | | 3 | 3 | 3 | 3 | 3 | 3 | 3 | 3 | 3 |

[Table 2]

| | Component name | Raw material name | Example | | | | | | | | |
|---|---|---|---|---|---|---|---|---|---|---|---|
| | | | 10 | 11 | 12 | 13 | 14 | 15 | 16 | 17 | 18 |
| A(a1) | Polyoxyethylene (30) sorbitol tetraoleate | HLB10.5: RHEODOL 430 (manufactured by Kao Corporation) | 3 | 5 | 5 | 5 | 6 | 6 | 5 | 3 | 5 |
| | Polyoxyethylene (20) sorbitan trioleate | HLB11: RHEODOL TW-O320V (manufactured by Kao Corporation) | | | | | | | | 2 | |
| | Polyoxyethylene (8) glyceryl isostearate | HLB10: EMALEX GWIS-108 (manufactured by Nihon Emulsion Co., Ltd.) | 3 | | | | | | | | |
| A(a2) | Polyoxyethylene (12) lauric acid ester | HLB14: EMANON 1112 (manufactured by Kao Corporation) | 10 | 5 | | | 7 | | 10 | 10 | 10 |
| | Polyoxyethylene (6) (Caprylate/Caprate) Glyceride | HLB15: TEGOSOFT GMC 6 MB (Evonik Operations GmbH) | | | 10 | | | | | | |
| | Polyoxyethylene (7) glyceryl monococoate | HLB13: LEVENOL C-301 (manufactured by Kao Corporation) | | 5 | | | | | | | |
| | Polyoxyethylene (9) lauryl ether | HLB13.6: EMULGEN 109P (manufactured by Kao Corporation) | | | | 10 | | | | | |
| | Polyoxyethylene (60) sorbitol tetraoleate | HLB13.8: RHEODOL 460 (manufactured by Kao Corporation) | | | | | 3 | 13 | | | |
| | Alkyl glucoside | HLB18: AG-10LK (manufactured by Kao Corporation) | 3 | 3 | | | 3 | | | 3 | |

(continued)

| | | Component name | Raw material name | Example | | | | | | | | |
|---|---|---|---|---|---|---|---|---|---|---|---|---|
| | | | | 10 | 11 | 12 | 13 | 14 | 15 | 16 | 17 | 18 |
| A(a3) | | Polyglyceryl-2 iso-stearate | HLB6: COS-MOL 41V (manufactured by The Nisshin Oillio Group, Ltd.) | 6 | 5 | 5 | 5 | 6 | 6 | | | |
| | | Sorbitan sesquialeate | HLB3.7: RHEODOL AO-15V (man-ufactured by Kao Corpora-tion) | | | | | | | 5 | | |
| | | Isostearyl glyceryl ether | HLB2: GE-IS (manufactured by Kao Corporation) | | | | | | | | 5 | |
| | | Sorbitan monooleate | HLB4.3: RHEODOL SP-O10V (manufactured by Kao Corporation) | | | | | | | | | 5 |
| B | | Light liquid isoparaffin | PARLEAM 4 (manufactured by NOF Corporation) | 23.5 | 24.2 | 25.3 | 25.3 | 23.5 | 23.5 | 25.3 | 24.2 | 25.3 |
| | | Isononyl isononanoate | SALACOS 99 (manufactured by The Nisshin Oillio Group, Ltd.) | 23.5 | 24.2 | 25.3 | 25.3 | 23.5 | 23.5 | 25.3 | 24.2 | 25.3 |
| | | Isopropyl myristate | EXCEPARL IPM (manufactured by Kao Corporation) | 23.5 | 24.2 | 25.3 | 25.3 | 23.5 | 23.5 | 25.3 | 24.2 | 25.3 |
| C | | Water | | 4.5 | 4.5 | 4 | 4 | 4.5 | 4.5 | 4 | 4.5 | 4 |
| | | Total | | 100 | 100 | 100 | 100 | 100 | 100 | 100 | 100 | 100 |
| | | Mixed HLB of component (A) | | 11.7 | 11.8 | 11.6 | 10.9 | 11.7 | 11.1 | 10.6 | 11.2 | 10.7 |
| | | (a2)/[(a1)+(a3)] | | 1.1 | 1.3 | 1.0 | 1.0 | 1.1 | 1.1 | 1.0 | 1.3 | 1.0 |
| | | Total amount of component (A) | | 25 | 23 | 20 | 20 | 25 | 25 | 20 | 23 | 20 |
| | | Total amount of oil phase (B) | | 70.5 | 72.5 | 76.0 | 76.0 | 70.5 | 70.5 | 76.0 | 72.5 | 76.0 |
| | | Arithmetic mean of viscosity of oil phase (B) | | 6.3 | 6.3 | 6.3 | 6.3 | 6.3 | 6.3 | 6.3 | 6.3 | 6.3 |
| | | C/A | | 0.18 | 0.20 | 0.20 | 0.20 | 0.18 | 0.18 | 0.20 | 0.20 | 0.20 |
| | | Appearance of composition (immediately after production, 25°C) | | 3 | 3 | 3 | 3 | 3 | 3 | 3 | 3 | 3 |
| | | Appearance of composition (50°C) | | 3 | 3 | 3 | 3 | 3 | 3 | 3 | 3 | 3 |
| | | Appearance of composition (-5°C) | | 3 | 3 | 3 | 3 | 3 | 3 | 3 | 3 | 3 |

(continued)

| | Component name | Raw material name | Example | | | | | | | | |
|---|---|---|---|---|---|---|---|---|---|---|---|
| | | | 10 | 11 | 12 | 13 | 14 | 15 | 16 | 17 | 18 |
| | Cleansing power when 100 g of friction force is applied | | 5 | 5 | 5 | 5 | 5 | 4 | 5 | 5 | 5 |
| | Cleansing power when 3 g of friction force is applied | | 5 | 5 | 3 | 3 | 5 | 3 | 4 | 4 | 3 |
| | Rinsing properties | | 3 | 3 | 3 | 3 | 3 | 2 | 3 | 3 | 3 |

[Table 3]

| | Component name | Raw material name | Example | | | | | | | |
|---|---|---|---|---|---|---|---|---|---|---|
| | | | 19 | 20 | 21 | 22 | 23 | 24 | 25 | 26 |
| A(a1) | Polvoxvethylene (30) sorbitol tetraoleate | HLB10.5: RHEODOL 430 (manufactured by Kao Corporation) | 6 | 6 | 6 | 6 | 3 | | 7 | 8 |
| | Polvoxvethylene (20) sorbitan trioleate | HLB11: RHEODOL TW-O320V (manufactured bv Kao Corporation) | | | | | | 10 | | |
| A(a2) | Polvoxvethylene (12) lauric acid ester | HLB14: EMANON 1112 (manufactured bv Kao Corporation) | 10 | 10 | 10 | 10 | 10 | 10 | 9 | 6 |
| | Alkyl glucoside | HLB18: AG-10LK (manufactured by Kao Corporation) | 3 | 3 | 3 | 3 | 3 | 3 | 3 | 3 |

(continued)

| | Component name | Raw material name | Example | | | | | | | |
|---|---|---|---|---|---|---|---|---|---|---|
| | | | 19 | 20 | 21 | 22 | 23 | 24 | 25 | 26 |
| A(a3) | Polyglyceryl-2 isostearate | HLB6: COSMOL 41V (manufactured bv The Nisshin Oillio Group, Ltd.) | 3 | 3 | | | 3 | 2 | 6 | 8 |
| | Isostearyl glyceryl ether | HLB2:GE-IS (manufactured by Kao Corporation) | | | | | | 6 | | |
| | Sorbitan tristearate | HLB2.1: RHEODOL SP-S30V (manufactured by Kao Corporation) | 3 | | 6 | | | | | |
| | Sorbitan trioleate | HLB1.8: RHEODOL SP-030V (manufactured by Kao Corporation) | | 3 | | 6 | | | | |
| B | Light liquid isoparaffin | PARLEAM 4 (manufactured bv NOF Corporation) | 23.5 | 23.5 | 23.5 | 23.5 | 23.7 | 23.7 | 23.5 | 23.5 |
| | Isononyl isononanoate | SALACOS 99 (manufactured bv The Nisshin Oillio Group, Ltd.) | 23.5 | 23.5 | 23.5 | 23.5 | 23.7 | 23.7 | 23.5 | 23.5 |
| | Isopropyl myristate | EXCEPARL IPM (manufactured by Kao Corporation) | 23.5 | 23.5 | 23.5 | 23.5 | 23.7 | 23.7 | 23.5 | 23.5 |
| C | Water | | 4.5 | 4.5 | 4.5 | 4.5 | 4 | 4 | 4.5 | 4.5 |
| | Total | | 100 | 100 | 100 | 100 | 100 | 100 | 100 | 100 |
| | Mixed HLB of component (A) | | 11.3 | 11.2 | 10.8 | 10.7 | 10.2 | 12.6 | 11.6 | 10.8 |
| | (a2)/[(a1)+(a3)] | | 1.1 | 1.1 | 1.1 | 1.1 | 1.1 | 1.1 | 0.92 | 0.56 |
| | Total amount of component (A) | | 25 | 25 | 25 | 25 | 25 | 25 | 25 | 25 |
| | Total amount of oil phase (B) | | 70.5 | 70.5 | 70.5 | 70.5 | 71.0 | 71.0 | 70.5 | 70.5 |
| | Arithmetic mean of viscosity of oil phase (B) | | 6.3 | 6.3 | 6.3 | 6.3 | 6.3 | 6.3 | 6.5 | 6.5 |
| | C/A | | 0.18 | 0.18 | 0.18 | 0.18 | 0.16 | 0.16 | 0.18 | 0.18 |
| | Appearance of composition (immediately after production, 25°C) | | 2 | 3 | 2 | 2 | 3 | 3 | 3 | 3 |
| | Appearance of composition (50°C) | | 3 | 3 | 1 | 1 | 3 | 2 | 3 | 3 |
| | Appearance of composition (-5°C) | | 1 | 3 | 1 | 1 | 3 | 1 | 3 | 3 |

(continued)

| | Component name | Raw material name | Example | | | | | | | |
|---|---|---|---|---|---|---|---|---|---|---|
| | | | 19 | 20 | 21 | 22 | 23 | 24 | 25 | 26 |
| | Cleansing power when 100 g of friction force is applied | | 5 | 5 | 5 | 5 | 4 | 5 | 4 | 3 |
| | Cleansing power when 3 g of friction force is applied | | 5 | 5 | 5 | 4 | 3 | 5 | 4 | 3 |
| | Rinsing properties | | 3 | 3 | 3 | 3 | 2 | 3 | 3 | 2 |

[Table 4]

| | Component name | Raw material name | Example | | Comparative Example | | | | | |
|---|---|---|---|---|---|---|---|---|---|---|
| | | | 27 | 28 | 1 | 2 | 3 | 4 | 5 | 6 |
| A(a1) | Polyoxyethylene (30) sorbitol tetraoleate | HLB10.5: RHEODOL 430 (manufactured by Kao Corporation) | 4.5 | 4 | 10 | 2.5 | 2.5 | 5 | | 5 |
| A(a2) | Polyoxyethylene (12) lauric acid ester | HLB14: EMANON 1112 (manufactured by Kao Corporation) | 10 | 10 | 2.5 | 10 | 2.5 | | 5 | 5 |
| | Polyoxyethylene (7) glyceryl monococoate | HLB13: LEVENOL C-301 (manufactured by Kao Corporation) | 3 | 4 | | | | | | |
| | Alkyl glucoside | HLB18: AG-10LK (manufactured by Kao Corporation) | 3 | 3 | 3 | 3 | 3 | | 3 | 3 |
| A(a3) | Polyglyceryl-2 isostearate | HLB6: COSMOL 41V (manufactured by The Nisshin Oillio Group, Ltd.) | 4.5 | 4 | 2.5 | 2.5 | 10 | 5 | 5 | |
| B | Light liquid isoparaffin | PARLEAM 4 (manufactured by NOF Corporation) | 23.5 | 23.5 | 25.8 | 25.8 | 25.8 | 28.5 | 27.5 | 27.5 |
| | Isononyl isononanoate | SALACOS 99 (manufactured bv The Nisshin Oillio Group, Ltd.) | 23.5 | 23.5 | 25.8 | 25.8 | 25.8 | 28.5 | 27.5 | 27.5 |
| | Isopropyl myristate | EXCEPARL IPM (manufactured by Kao Corporation) | 23.5 | 23.5 | 25.8 | 25.8 | 25.8 | 28.5 | 27.5 | 27.5 |

(continued)

|  | Component name | Raw material name | Example 27 | Example 28 | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|---|---|---|
| C | Water |  | 4.5 | 4.5 | 4.5 | 4.5 | 4.5 | 4.5 | 4.5 | 4.5 |
|  | Total |  | 100 | 100 | 100 | 100 | 100 | 100 | 100 | 100 |
|  | Mixed HLB of component (A) |  | 12.3 | 12.5 | 11.6 | 13.1 | 9.7 | 8.3 | 11.8 | 13.6 |
|  | (a2)/[(a1)+(a3)] |  | 1.78 | 2.13 | 0.44 | 2.60 | 0.44 | 0.00 | 1.60 | 1.60 |
|  | Total amount of component (A) |  | 25 | 25 | 18 | 18 | 18 | 10 | 13 | 13 |
|  | Total amount of oil phase (B) |  | 70.5 | 70.5 | 77.5 | 77.5 | 77.5 | 85.5 | 82.5 | 82.5 |
|  | Arithmetic mean of viscosity of oil phase (B) |  | 6.5 | 6.5 | 6.5 | 6.5 | 6.5 | 6.3 | 6.3 | 6.3 |
|  | C/A |  | 0.18 | 0.18 | 0.25 | 0.25 | 0.25 | 0.45 | 0.35 | 0.35 |
|  | Appearance of composition (immediately after production, 25°C) |  | 3 | 3 | 3 | 1 | 3 | 1 | 1 | 1 |
|  | Appearance of composition (50°C) |  | 3 | 3 | 3 | - | 3 | - | - | - |
|  | Appearance of composition (-5°C) |  | 2 | 1 | 1 | - | 3 | - | - | - |
|  | Cleansing power when 100 g of friction force is applied |  | 5 | 5 | 5 | 5 | 2 | 4 | 5 | 4 |
|  | Cleansing power when 3 g of friction force is applied |  | 3 | 3 | 3 | 3 | 1 | 2 | 1 | 3 |
|  | Rinsing properties |  | 3 | 3 | 1 | - | 1 | - | - | - |

[Table 5]

|  | Component name | Raw material name | Ex. 29 | Ex. 30 | Ex. 31 | Ex. 32 | Ex. 33 | Ex. 34 | Comp. Ex. 7 | Comp. Ex. 8 | Comp. Ex. 9 | Comp. Ex. 10 |
|---|---|---|---|---|---|---|---|---|---|---|---|---|
| A(a1) | Polyoxyethylene (30) sorbitol tetraoleate | HLB10.5: RHEODOL 430 (manufactured by Kao Corporation) | 5 | 5 | 5 | 5 | 5 | 5 | 5 | 5 | 5 | 5 |
| A(a2) | Polyoxyethylene (12) lauric acid ester | HLB14: EMANON 1112 (manufactured by Kao Corporation) | 8 | 8 | 8 | 8 | 8 | 8 | 8 | 8 | 8 | 8 |
|  | Alkyl glucoside | HLB18: MYDOL 10 (manufactured by Kao Corporation) active | 3 | 3 | 3 | 3 | 3 | 3 | 3 | 3 | 3 | 3 |
| A(a3) | Polyglyceryl-2 isostearate | HLB6: COSMOL 41V (manufactured by The Nisshin Oillio Group, Ltd.) | 5 | 5 | 5 | 5 | 5 | 5 | 5 | 5 | 5 | 5 |

(continued)

| | | Component name | Raw material name | Example | | | | | | Comparative Example | | | |
|---|---|---|---|---|---|---|---|---|---|---|---|---|---|
| | | | | 29 | 30 | 31 | 32 | 33 | 34 | 7 | 8 | 9 | 10 |
| B | | Isododecane | Marukasol R (manufactured by Maruzen Petrochemical) | 5 | 5 | | | | | | | | |
| | | Isohexadecane | Permethyl 101A (manufactured bv Nihon Koken Kogyo Co., Ltd.) | 7.5 | 7.5 | | | | | | | | |
| | | Light liquid isoparaffin | PARLEAM 4 (manufactured bv NOF Corporation) | 15 | 16 | 24 | 15 | 15 | 8 | 16 | | 8 | |
| | | Isopropyl myristate | EXCEPARL IPM (manufactured by Kao Corporation) | 15 | 15 | 25.5 | 15 | 15 | 8 | 17 | | 8 | |
| | | Isononyl isononanoate | SALACOS 99 (manufactured bv The Nisshin Oillio Group, Ltd.) | 17 | 26 | 25 | 17 | 17 | 9 | 15 | | 9 | |
| | | Liquid paraffin | HICALL K-140 (manufactured by Kaneda Co., Ltd.) | 5 | | | | | | | | | |
| | | Liquid paraffin | HICALL K-160 (manufactured by Kaneda Co., Ltd.) | 5 | | | | | | | | | |
| | | Cetyl ethylhexanoate | EXCEPARL HO (manufactured by Kao Corporation) | 5 | 5 | | | | | | | | |
| | | Liquid isoparaffin | PARLEAM EX (manufactured bv NOF Corporation) | | | | 27.5 | | 49.5 | | 74.5 | | |
| | | Liquid paraffin | HICALL 230 (manufactured by Kaneda Co., Ltd.) | | | | | 27.5 | | | | | 75.5 |
| | | Caprylic/capric triglyceride | COCONARD MT (manufactured by Kao Corporation) | | | | | | | 26.5 | | 49.5 | |
| C | | Water | Water | 4.5 | 4.5 | 4.5 | 4.5 | 4.5 | 4.5 | 4.5 | 4.5 | 4.5 | 4.5 |
| | | Total | | 100 | 100 | 100 | 100 | 100 | 100 | 100 | 100 | 100 | 100 |
| | | Mixed HLB of component (A) | | 11.5 | 11.5 | 11.5 | 11.5 | 11.5 | 11.5 | 11.8 | 11.5 | 11.5 | 11.5 |

(continued)

| Component name | Raw material name | Example | | | | | | Comparative Example | | | |
|---|---|---|---|---|---|---|---|---|---|---|---|
| | | 29 | 30 | 31 | 32 | 33 | 34 | 7 | 8 | 9 | 10 |
| (a2)/[(a1)+(a3)] | | 1.1 | 1.1 | 1.1 | 1.1 | 1.1 | 1.1 | 1.1 | 1.1 | 1.1 | 1.1 |
| Total amount of component (A) | | 21 | 21 | 21 | 21 | 21 | 21 | 21 | 21 | 21 | 21 |
| Total amount of oil phase (B) | | 74.5 | 74.5 | 74.5 | 74.5 | 74.5 | 74.5 | 74.5 | 74.5 | 74.5 | 75.5 |
| Arithmetic mean of viscosity of oil phase (B) | | 6.6 | 6.5 | 6.3 | 9.2 | 10.6 | 11.4 | 12.2 | 14.0 | 17.4 | 18.0 |
| C/A | | 0.21 | 0.21 | 0.21 | 0.21 | 0.21 | 0.21 | 0.21 | 0.21 | 0.21 | 0.21 |
| Appearance of composition (immediately after preparation, 25°C) | | 3 | 3 | 3 | 3 | 3 | 3 | 3 | 1 | 1 | 1 |
| Appearance of composition (-5°C) | | 3 | 3 | 3 | 3 | 1 | 1 | 1 | - | - | - |
| Cleansing power when 100 g of friction force is applied | | 5 | 5 | 5 | 5 | 5 | 4 | 3 | 3 | 2 | 1 |
| Cleansing power when 3 g of friction force is applied | | 5 | 5 | 5 | 4 | 3 | 3 | 1 | 1 | 1 | 1 |
| Rinsing properties | | 3 | 3 | 3 | 3 | 3 | 3 | 3 | - | - | - |

[Table 6]

| | Component name | Raw material name | Comparative Example 11 | Example 35 | Example 36 | Example 37 | Example 38 | Comparative Example 12 | Example 39 | Example 40 |
|---|---|---|---|---|---|---|---|---|---|---|
| A(a1) | Polyoxyethylene (30) sorbitol tetraoleate | HLB10.5: RHEODOL 430 (manufactured by Kao Corporation) | 2 | 3 | 4.2 | 7.5 | 9 | 11.4 | 7.5 | 10.0 |
| A(a2) | Polyoxyethylene (12) lauric acid ester | HLB14: EMANON 1112 (manufactured by Kao Corporation) | 3.3 | 5 | 7 | 12.5 | 15 | 19 | 15 | 20.0 |
| | Alkyl glucoside | HLB18: MYDOL 10 (manufactured by Kao Corporation) active | 1 | 1.5 | 2.1 | 3.75 | 4.5 | 5.7 | | |
| A(a3) | Polyglyceryl-2 isostearate | HLB6: COSMOL 41V (manufactured by The Nisshin Oillio Group, Ltd.) | 2 | 3 | 4.2 | 7.5 | 9 | 11.4 | 7.5 | 10.0 |
| B | Light liquid isoparaffin | PARLEAM 4 (manufactured by NOF Corporation) | 30.1 | 28.4 | 26.5 | 21.0 | 18.6 | 14.7 | 19.3 | 19.6 |
| | Isononyl isononanoate | SALACOS 99 (manufactured by The Nisshin Oillio Group, Ltd.) | 30.1 | 28.4 | 26.5 | 21.0 | 18.6 | 14.7 | 19.3 | 19.6 |
| | Isopropyl myristate | EXCEPARL IPM (manufactured by Kao Corporation) | 30.1 | 28.4 | 26.5 | 21.0 | 18.6 | 14.7 | 19.3 | 19.6 |
| C | Water | | 1.5 | 2.3 | 3.2 | 5.6 | 6.8 | 8.6 | 12.0 | 1.2 |
| | Total | | 100.0 | 100.0 | 100.0 | 100.0 | 100.0 | 100.0 | 100.0 | 100.0 |
| | Mixed HLB of component (A) | | 11.7 | 11.7 | 11.7 | 11.7 | 11.7 | 11.7 | 11.6 | 11.6 |
| | (a2)/[(a1)+(a3)] | | 1.1 | 1.1 | 1.1 | 1.1 | 1.1 | 1.1 | 1.1 | 1.1 |
| | Total amount of component (A) | | 8.3 | 12.5 | 17.5 | 31.3 | 37.5 | 47.5 | 30.0 | 40.0 |
| | Total amount of oil phase (B) | | 90.2 | 85.3 | 79.4 | 63.1 | 55.8 | 44.0 | 58.0 | 58.8 |
| | Arithmetic mean of viscosity of oil phase (B) | | 6.5 | 6.5 | 6.5 | 6.5 | 6.5 | 6.5 | 6.5 | 6.5 |
| | C/A | | 0.18 | 0.18 | 0.18 | 0.18 | 0.18 | 0.18 | 0.40 | 0.03 |
| | Appearance of composition (immediately after production, 25°C) | | 1 | 3 | 3 | 3 | 3 | 3 | 3 | 2 |
| | Appearance of composition (50°C) | | - | 3 | 3 | 3 | 3 | 3 | 3 | 2 |
| | Appearance of composition (-5°C) | | - | 2 | 3 | 3 | 3 | 3 | 2 | 1 |

(continued)

| Component name | Raw material name | Comparative Example | Example | | | | Comparative Example | Example | |
|---|---|---|---|---|---|---|---|---|---|
| | | 11 | 35 | 36 | 37 | 38 | 12 | 39 | 40 |
| Cleansing power when 100 g of friction force is applied | | 5 | 5 | 5 | 5 | 5 | 5 | 4 | 5 |
| Cleansing power when 3 g of friction force is applied | | 2 | 3 | 4 | 5 | 3 | 2 | 3 | 4 |
| Cleansing power without friction force | | 1 | 1 | 3 | 5 | 3 | 2 | 2 | 4 |
| Rinsing properties | | - | 3 | 3 | 3 | 3 | 2 | 3 | 3 |

EP 4 613 258 A1

[Table 7]

| | Component name | Raw material name | Comparative Example | Example | | | | | Comparative Example | |
|---|---|---|---|---|---|---|---|---|---|---|
| | | | 13 | 41 | 42 | 43 | 44 | 45 | 14 | 15 |
| A(a1) | Polvoxvethylene (30) sorbitol tetraoleate | HLB10.5: RHEODOL 430 (manufactured bv Kao Corporation) | 5 | 5 | 5 | 5 | 5 | 5 | 5 | 3 |
| A(a2) | Polvoxvethylene (12) lauric acid ester | HLB14: EMANON 1112 (manufactured bv Kao Corporation) | 10 | 10 | 10 | 10 | 10 | 10 | 10 | 11 |
| | Alkyl glucoside | HLB18: AG-10LK (man-ufactured bv Kao Corpora-tion) | | | | | | | | 3 |
| A(a3) | Polyglyceryl-2 isostearate | HLB6: COSMOL 41V (manufactured by The Nisshin Oillio Group, Ltd.) | 5 | 5 | 5 | 5 | 5 | 5 | 5 | 5 |
| B | Isododecane | Marukasol R (manufactured bv Maruzen Petrochemical) | | | | | | | | 6 |
| | Light liquid isoparaffin | PARLEAM 4 (manufactured bv NOF Corporation) | 26.7 | 26.3 | 26.0 | 25.3 | 24.3 | 24.2 | 23.8 | |
| | Isononyl isononanoate | SALACOS 99 (manufactured bv The Nisshin Oillio Group, Ltd.) | 26.7 | 26.3 | 26.0 | 25.3 | 24.3 | 24.2 | 23.8 | |
| | Isopropyl myris-tate | EXCEPARL IPM (manufac-tured bv Kao Corporation) | 26.7 | 26.3 | 26.0 | 25.3 | 24.3 | 24.2 | 23.8 | 8 |
| | Liquid isoparaffin | PARLEAM EX (manufactured hv NOF Cor-poration) | | | | | | | | 2 |

(continued)

| | Component name | Raw material name | Comparative Example 13 | Example 41 | 42 | 43 | 44 | 45 | Comparative Example 14 | 15 |
|---|---|---|---|---|---|---|---|---|---|---|
| C | Water | | 0 | 1 | 2 | 4 | 7 | 7.5 | 8.5 | 27 |
| | 1,3-Butylene glycol | 1.3-BG-P (manufactured bv KH Neochem Co., Ltd.) | | | | | | | | 20 |
| | Sorbitol | Sorbitol #650 (manufactured bv Kao Corporation) | | | | | | | | 15 |
| | Total | | 100 | 100 | 100 | 100 | 100 | 100 | 100 | 100 |
| | Mixed HLB of component (A) | | 11.1 | 11.1 | 11.1 | 11.1 | 11.1 | 11.1 | 11.1 | 11.1 |
| | (a2)/[(a1)+(a3)] | | 1.0 | 1.0 | 1.0 | 1.0 | 1.0 | 1.0 | 1.0 | 1.75 |
| | Total amount of component (A) | | 20 | 20 | 20 | 20 | 20 | 20 | 20 | 22 |
| | Total amount of oil phase (B) | | 80.0 | 79.0 | 78.0 | 76.0 | 73.0 | 72.5 | 71.5 | 16.0 |
| | Arithmetic mean of viscosity of oil phase (B) | | 6.3 | 6.3 | 6.3 | 6.3 | 6.3 | 6.3 | 6.3 | 6.2 |
| | C/A | | 0.00 | 0.05 | 0.10 | 0.20 | 0.35 | 0.38 | 0.43 | 2.82 |
| | Appearance of composition (immediately after production, 25°C) | | 1 | 2 | 3 | 3 | 2 | 2 | 1 | 3 |
| | Appearance of composition (50°C) | | - | 3 | 3 | 3 | 3 | 3 | - | 1 |
| | Appearance of composition (-5°C) | | - | 2 | 2 | 3 | 1 | 1 | - | 2 |
| | Cleansing power when 100 g of friction force is applied | | 5 | 5 | 5 | 5 | 5 | 5 | 5 | 3 |
| | Cleansing power when 3 g of friction force is applied | | 2 | 3 | 4 | 5 | 5 | 4 | 1 | 1 |
| | Cleansing power without friction force | | 2 | 3 | 4 | 5 | 3 | 2 | 1 | 1 |
| | Rinsing properties | | - | 3 | 3 | 3 | 3 | 3 | - | 3 |

[Table 8]

| | Component name | Raw material name | Comparative Example 16 | Example 46 | 47 | 48 | 49 | 50 | Comparative Example 17 |
|---|---|---|---|---|---|---|---|---|---|
| A(a1) | Polvoxvethylene (30) sorbitol tetraoleate | HLB10.5: RHEODOL 430 (manufactured bv Kao Corporation) | 15 | 12.5 | 10 | 6 | 5 | 3.5 | 3 |
| A(a2) | Polvoxvethylene (12) lauric acid ester | HLB14: EMANON 1112 (manufactured bv Kao Corporation) | 30 | 25 | 20 | 12 | 10 | 7 | 6 |

(continued)

| | Component name | Raw material name | Comparative Example 16 | Example 46 | Example 47 | Example 48 | Example 49 | Example 50 | Comparative Example 17 |
|---|---|---|---|---|---|---|---|---|---|
| A(a3) | Polyglyceryl-2 isostearate | HLB6: COSMOL 41V (manufactured by The Nisshin Oillio Group, Ltd.) | 15 | 12.5 | 10 | 6 | 5 | 3.5 | 3 |
| B | Light liquid isoparaffin | PARLEAM 4 (manufactured bv NOF Corporation) | 11.7 | 15.0 | 18.3 | 23.7 | 25.0 | 27.0 | 27.7 |
| | Isononyl isononanoate | SALACOS 99 (manufactured bv The Nisshin Oillio Group, Ltd.) | 11.7 | 15.0 | 18.3 | 23.7 | 25.0 | 27.0 | 27.7 |
| | Isopropyl myristate | EXCEPARL IPM (manufactured bv Kao Corporation) | 11.7 | 15.0 | 18.3 | 23.7 | 25.0 | 27.0 | 27.7 |
| C | Water | | 5 | 5 | 5 | 5 | 5 | 5 | 5 |
| | Total | | 100 | 100 | 100 | 100 | 100 | 100 | 100 |
| | Mixed HLB of component (A) | | 11.1 | 11.1 | 11.1 | 11.1 | 11.1 | 11.1 | 11.1 |
| | (a2)/[(a1)+(a3)] | | 1.0 | 1.0 | 1.0 | 1.0 | 1.0 | 1.0 | 1.0 |
| | Total amount of component (A) | | 60 | 50 | 40 | 24 | 20 | 14 | 12 |
| | Total amount of oil phase (B) | | 35.0 | 45.0 | 55.0 | 71.0 | 75.0 | 81.0 | 83.0 |
| | Arithmetic mean of viscosity of oil phase (B) | | 6.3 | 6.3 | 6.3 | 6.3 | 6.3 | 6.3 | 6.3 |
| | C/A | | 0.08 | 0.10 | 0.13 | 0.21 | 0.25 | 0.36 | 0.42 |
| | Appearance of composition (immediately after production, 25°C) | | 1 | 2 | 3 | 3 | 3 | 3 | 2 |
| | Appearance of composition (50°C) | | - | 2 | 2 | 3 | 3 | 3 | 2 |
| | Appearance of composition (-5°C) | | - | 1 | 2 | 3 | 3 | 2 | 2 |
| | Cleansing power when 100 g of friction force is applied | | 2 | 4 | 5 | 5 | 5 | 4 | 2 |
| | Cleansing power when 3 g of friction force is applied | | 2 | 3 | 4 | 5 | 5 | 3 | 2 |
| | Cleansing power without friction force | | 1 | 1 | 4 | 5 | 4 | 3 | 2 |
| | Rinsing properties | | - | 3 | 3 | 3 | 3 | 3 | 3 |

[Table 9]

| | Component name | Raw material name | Comparative Example | Example | | | | | | |
|---|---|---|---|---|---|---|---|---|---|---|
| | | | 18 | 51 | 52 | 53 | 54 | 55 | 56 | 57 |
| A(a1) | Polyoxyethylene (30) sorbitol tetraoleate | HLB10.5: RHEODOL 430 (manufactured by Kao Corporation) | 6 | 6 | 6 | 6 | 6 | 6 | 6 | |
| | Polvoxvethylene (6) sorbitan oleate | HLB10: RHEODOL TW-O106V (manufactured bv Kao Corporation) | | | | | | | | 6 |
| A(a2) | Polvoxvethylene (12) lauric acid ester | HLB14: EMANON 1112 (manufactured bv Kao Corporation) | 10 | 10 | 10 | 14 | 14 | 14 | 10 | 10 |
| | Polyoxyethylene (7) glyceryl monococoate | HLB13: LEVENOL C-301 (manufactured by Kao Corporation) | 4 | 4 | 4 | 0 | 0 | 0 | 4 | 4 |
| A(a3) | Polyglyceryl-2 isostearate | HLB6: COSMOL 41V (manufactured bv The Nisshin Oillio Group, Ltd.) | 6 | 6 | 6 | 6 | 6 | 6 | 6 | 6 |
| B | Light liquid isoparaffin | PARLEAM 4 (manufactured bv NOF Corporation) | 24.7 | 22.7 | 22.7 | 22.7 | 22.7 | 22.7 | 22.7 | 22.7 |
| | Isononyl isononanoate | SALACOS 99 (manufactured bv The Nisshin Oillio Group, Ltd.) | 24.7 | 22.7 | 22.7 | 22.7 | 22.7 | 22.7 | 22.7 | 22.7 |
| | Isopropyl myristate | EXCEPARL IPM (manufactured bv Kao Corporation) | 24.7 | 22.7 | 22.7 | 22.7 | 22.7 | 22.7 | 22.7 | 22.7 |

(continued)

| | Component name | Raw material name | Comparative Example 18 | Example 51 | 52 | 53 | 54 | 55 | 56 | 57 |
|---|---|---|---|---|---|---|---|---|---|---|
| C | Water | | 0 | 6 | | | | 5 | 4 | 6 |
| | Ethanol | Ethanol (Japanese Pharmacopoeia, fermentation) (manufactured by Japan Alcohol Corporation) | | | 6 | | | | | |
| | 1,3-Butvlene glycol | 1.3-BG-P (manufactured bv KH Neochem Co., Ltd.) | | | | 6 | | 0.5 | 1 | |
| | Dipropylene glycol | Propylene glycol for cosmetic (manufactured bv ADEKA) | | | | | 6 | 0.5 | | |
| | Glycerol | Glycerol 99.5% (USP) (manufactured bv IOI Acidchem Sdn.Bhd) | | | | | | | 0.5 | |
| | Sorbitol | Sorbitol #650 (manufactured hv Kao Corporation) | | | | | | | 0.5 | |
| | Total | | 100 | 100 | 100 | 100 | 100 | 100 | 100 | 100 |
| | Mixed HLB of component (A) | | 11.1 | 11.1 | 11.1 | 11.3 | 11.3 | 11.3 | 11.1 | 11.1 |
| | (a2)/[(a1)+(a3)] | | 1.2 | 1.2 | 1.2 | 1.2 | 1.2 | 1.2 | 1.2 | 1.2 |
| | Total amount of component (A) | | 26 | 26 | 26 | 26 | 26 | 26 | 26 | 26 |
| | Total amount of oil phase (B) | | 74.0 | 68.0 | 68.0 | 68.0 | 68.0 | 68.0 | 68.0 | 68.0 |
| | Arithmetic mean of viscosity of oil phase (B) | | 6.3 | 6.3 | 6.3 | 6.3 | 6.3 | 6.3 | 6.3 | 6.3 |
| | C/A | | 0.00 | 0.23 | 0.23 | 0.23 | 0.23 | 0.23 | 0.23 | 0.23 |
| | Appearance of composition (immediately after production, 25°C) | | 1 | 3 | 2 | 2 | 2 | 3 | 3 | 3 |
| | Appearance of composition (50°C) | | - | 3 | 2 | 2 | 2 | 3 | 2 | 3 |
| | Appearance of composition (-5°C) | | - | 3 | 2 | 2 | 2 | 3 | 2 | 3 |
| | Cleansing power when 100 g of friction force is applied | | 5 | 5 | 5 | 5 | 5 | 5 | 5 | 5 |
| | Cleansing power when 3 g of friction force is applied | | 3 | 5 | 5 | 5 | 5 | 5 | 5 | 5 |
| | Cleansing power without friction force | | 2 | 5 | 5 | 5 | 4 | 5 | 5 | 3 |
| | Rinsing properties | | - | 3 | 3 | 3 | 3 | 3 | 3 | 3 |

Formulation Example 1

A cleansing composition with the composition shown in Table 10 was produced in the same manner as in Examples 1 to 57, and the appearance of the composition (immediately after production, 25°C), the appearance of the composition (-5°C), the cleansing power and the rinsing properties were evaluated. The results are shown together in Table 10.

[Table 10]

|  | | Component name | Raw material name | Formulation Example 1 |
|---|---|---|---|---|
| A(a1) | | Polyoxyethylene (30) sorbitol tetraoleate | (manufactured by Kao Corporation) | 5 |
| | | Polyoxyethylene (20) sorbitan trioleate | HLB11: RHEODOL TW-O320V (manufactured by Kao Corporation) | 1 |
| A(a2) | | Polyoxyethylene (12) lauric acid ester | HLB14: EMANON 1112 (manufactured by Kao Corporation) | 8 |
| | | Polyoxyethylene (7) glyceryl monococoate | HLB13: LEVENOL C-301 (manufactured by Kao Corporation) | 2 |
| | | Alkyl glucoside | HLB 18: MYDOL 10LK (manufactured by Kao Corporation) active | 3 |
| A(a3) | | Polyglyceryl-2 isostearate | HLB6: COSMOL 41V (manufactured by The Nisshin Oillio Group, Ltd.) | 6 |
| B | | Isododecane | Marukasol R (manufactured by Maruzen Petrochemical) | 5 |
| | | Light liquid isoparaffin | PARLEAM 4 (manufactured by NOF Corporation) | 17.5 |
| | | Isopropyl myristate | EXCEPARL IPM (manufactured by Kao Corporation) | 19 |
| | | Isononyl isononanoate | SALACOS 99 (manufactured by The Nisshin Oillio Group, Ltd.) | 18 |
| | | Liquid paraffin | HICALL K-160 (manufactured by Kaneda Co., Ltd.) | 5 |
| | | Cetyl ethylhexanoate | EXCEPARL HO (manufactured by Kao Corporation) | 5 |
| C | | Water | | 4.5 |
| | | 1.3-Butylene glycol | 1.3-BG-P (manufactured by KH Neochem Co., Ltd.) | 1 |
| | Total | | | 100 |
| | Mixed HLB of component (A) | | | 11.7 |
| | (a2)/[(a1)+{a3)] | | | 1.2 |
| | Total amount of component (A) | | | 25 |
| | Total amount of oil phase (B) | | | 69.5 |
| | Arithmetic mean of viscosity of oil phase (B) | | | 6.6 |
| | C/A | | | 0.22 |
| | Appearance of composition (immediately after production, 25°C) | | | 3 |
| | Appearance of composition (-5°C) | | | 3 |
| | Cleansing power when 100 g of friction force is applied | | | 5 |
| | Cleansing power when 3 g of friction force is applied | | | 5 |
| | Cleansing power without friction force | | | 5 |

(continued)

| | Component name | Raw material name | Formulation Example 1 |
|---|---|---|---|
| | Rinsing properties | | 3 |

**Claims**

1. A cleansing composition comprising the following components (A), (B) and (C):

    (A) from 9 to 50% by mass of a nonionic surfactant comprising:

        (a1) a nonionic surfactant having an HLB value of 8.5 or more and less than 12.5,
        (a2) a nonionic surfactant having an HLB value of 12.5 or more and 18 or less, and
        (a3) a nonionic surfactant having an HLB value of 1.8 or more and less than 8.5,
        wherein a mass ratio of the component (a2) to the total amount of the components (a1) and (a3), (a2)/[(a1)+(a3)], is from 0.5 to 2.5,

    (B) from 45 to 90% by mass of an oil phase having a viscosity at 25°C of 12.0 mPa·s or less, and
    (C) from 0.05 to 15% by mass of one or more selected from the group consisting of water, an alcohol having 2 to 3 carbon atoms and a polyol,

    wherein a mass ratio of the component (C) to the component (A), (C)/(A) is 0.40 or less.

2. The cleansing composition according to claim 1, wherein the component (A) has a mixed HLB value of from 10 to 13.

3. The cleansing composition according to claim 1 or 2, wherein a mass ratio of the component (C) to the component (A), (C)/(A) is 0.02 or more.

4. The cleansing composition according to any one of claims 1 to 3, wherein a content of the component (a1) is from 2 to 20% by mass in the entire composition.

5. The cleansing composition according to any one of claims 1 to 4, wherein a content of the component (a2) is from 5 to 30% by mass in the entire composition.

6. The cleansing composition according to any one of claims 1 to 5, wherein a content of the component (a3) is from 1 to 15% by mass in the entire composition.

7. The cleansing composition according to any one of claims 1 to 6, wherein the component (a1) is one or more selected from the group consisting of a POE sorbitan fatty acid ester, a POE sorbitol fatty acid ester, a POE glycerol fatty acid ester and a POE fatty acid ester.

8. The cleansing composition according to any one of claims 1 to 7, wherein the component (a2) is one or more selected from the group consisting of a POE fatty acid ester, a POE alkyl ether, a POE sorbitol fatty acid ester, a POE glycerol fatty acid ester, a polyglycerol fatty acid ester and an alkyl glucoside.

9. The cleansing composition according to any one of claims 1 to 8, wherein the component (a3) is one or more selected from the group consisting of a glycerol fatty acid ester, a polyglycerol fatty acid ester, an alkyl glyceryl ether and a sorbitan fatty acid ester.

10. The cleansing composition according to any one of claims 1 to 9, wherein the component (C) is one or more selected from the group consisting of water, ethanol, 1,3-butylene glycol and dipropylene glycol.

## INTERNATIONAL SEARCH REPORT

International application No.

**PCT/JP2023/038903**

| A. | CLASSIFICATION OF SUBJECT MATTER |
|---|---|

*A61K 8/34*(2006.01)i; *A61Q 1/14*(2006.01)i; *C11D 1/66*(2006.01)i; *C11D 1/68*(2006.01)i; *C11D 3/20*(2006.01)i
FI: A61K8/34; A61Q1/14; C11D1/66; C11D1/68; C11D3/20

According to International Patent Classification (IPC) or to both national classification and IPC

| B. | FIELDS SEARCHED |
|---|---|

Minimum documentation searched (classification system followed by classification symbols)

A61K8/34; A61Q1/14; C11D1/66; C11D1/68; C11D3/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2012-180305 A (KAO CORP) 20 September 2012 (2012-09-20) entire text | 1-10 |
| A | JP 2010-508323 A (UNILEVER N.V) 18 March 2010 (2010-03-18) entire text | 1-10 |

☐ Further documents are listed in the continuation of Box C.      ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **06 December 2023** | **16 January 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

## INTERNATIONAL SEARCH REPORT
### Information on patent family members

International application No.

**PCT/JP2023/038903**

| Patent document cited in search report | | | Publication date (day/month/year) | Patent family member(s) | Publication date (day/month/year) |
|---|---|---|---|---|---|
| JP | 2012-180305 | A | 20 September 2012 | (Family: none) | |
| JP | 2010-508323 | A | 18 March 2010 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018508484 A
- JP 2008184415 A

- JP 2009235001 A